# EUROPEAN PATENT SPECIFICATION

(11) **EP 3 595 751 B1**
(45) Date of publication and mention of the grant of the patent: **27.01.2021**
(21) Application number: 18709053.5
(22) Date of filing: 14.03.2018
(51) Int. Cl.: A61M 5/24, A61M 5/32, A61M 5/31

(54) **CAP ASSEMBLY WITH CARTRIDGE**
ARZNEIMITTELABGABEANORDNUNG MIT KARTUSCHENEINHEIT
ENSEMBLE D'ADMINISTRATION DE MÉDICAMENT AVEC UNITÉ DE CARTOUCHE

(30) Priority: 14.03.2017 EP 17160804
(43) Date of publication of application: 22.01.2020
(73) Proprietor: Novo Nordisk A/S, 2880 Bagsværd (DK)
(72) Inventor: LARSEN, André, 2880 Bagsværd (DK)
(86) International application number: PCT/EP2018/056369
(87) International publication number: WO 2018/167137

(56) References cited:
- EP-A1- 2 455 121
- WO-A1-2010/090747
- WO-A1-2015/055592
- WO-A1-2015/140262
- WO-A1-2015/165757
- WO-A1-2016/102299

## Description

The present invention generally relates to drug delivery devices adapted to expel a dose from a drug filled cartridge as well as components therefor.

### BACKGROUND OF THE INVENTION

In the disclosure of the present invention reference is mostly made to the treatment of diabetes, however, this is only an exemplary use of the present invention.

Drug delivery devices for subcutaneous injection of a fluid drug formulation via a hollow needle have greatly improved the lives of patients who must self-administer drugs and biological agents. Drug delivery devices may take many forms, including simple disposable devices that are little more than an ampoule with an injection means to relatively complex pre-filled disposable devices which may even be spring-driven, or they may be durable devices adapted to be used with pre-filled cartridges. Regardless of their form and type, they have proven to be great aids in assisting patients to self-administer injectable drugs and biological agents. They also greatly assist care givers in administering injectable medicines to those incapable of performing self-injections.

To further improve convenience and user-friendliness it has been proposed to provide drug delivery devices wherein the same subcutaneous needle can be used for multiple successive injections and automatically cleaned between injections. For example, WO 2015/062845 discloses a drug delivery device in which a small amount of a preservative-containing fluid drug formulation is transferred from the cartridge to a sealed reservoir in which the distal needle portion is "parked" when not in use. The reservoir comprises a distal needle-penetratable septum and is housed in an axially moveable "telescoping" shield assembly which can be moved proximally, this allowing the distal end portion of the needle to protrude through the septum during use of the device. After use the shield assembly is returned to its distal position with the needle distal portion arranged in the reservoir. Before initial use of the device the needle proximal end is arranged distally of the needle-penetratable cartridge septum and thus without flow communication therewith. During priming the needle is inserted in the cartridge and an amount of fluid drug is transferred to the reservoir. WO 2016/041883 discloses a prefilled drug delivery device (i.e. a fully disposable device comprising a non-replaceable cartridge) provided with such a telescoping reservoir assembly.

Although a fully disposable drug delivery device may be considered convenient in use, the actual design of such a device will normally be the result of a compromise between price and the quality of the design. Although disposable drug delivery devices presently on the market can be considered both safe and reliable, the perceived quality of such a device will normally be lower than for a re-useable ("durable") device adapted to be used with a replaceable cartridge, just as the price per cartridge normally will be higher as a durable device can be used for several years and be refilled several hundred times, this also providing an environmental advantage producing less waste.

However, the use of durable devices is more complicated, even though manufacturers of durable drug delivery devices continuously develop new products in which the replacing and changing of cartridges and needles are made easier. Disposable devices eliminate the need of changing cartridge and may be fitted with reusable needles, since the lack of option of refilling the device limits the number of times a reusable needle can be used.

Hence, the most cost-efficient treatment will be using a durable device, while the simplest treatment will be with a disposable device. It would be desirable to combine the two and be able to provide a simple and cost efficient solution to enable a simpler-to-use treatment more affordable and thereby enable more people in need the best possible treatment.

Further, performing the necessary insulin injection at the right time and in the right size is essential for managing diabetes, i.e. compliance with the specified insulin regimen is important. In order to make it possible for medical personnel to determine the effectiveness of a prescribed dosage pattern, diabetes patients are encouraged to keep a log of the size and time of each injection. However, such logs are normally kept in handwritten notebooks, from the logged information may not be easily uploaded to a computer for data processing. Furthermore, as only events, which are noted by the patient, are logged, the note book system requires that the patient remembers to log each injection, if the logged information is to have any value in the treatment of the patient's disease. A missing or erroneous record in the log results in a misleading picture of the injection history and thus a misleading basis for the medical personnel's decision making with respect to future medication. Accordingly, it may be desirable to automate the logging of delivery information from medication delivery systems.

Correspondingly, a number of drug delivery devices with a dose monitoring/acquisition feature has been provided, see e.g. in US 2009/0318865, WO 2010/052275, US 7,008,399 and WO 2014/187814. These devices are all of the durable type as it is currently considered too expensive to incorporate a monitoring feature in a pre-filled disposable drug delivery device. In this way the added costs due to the added electronic monitoring functionality can be divided out on the doses given using a durable device such that in durable devices the added costs pr. dose given is fairly small, but in a disposable device will be significant.

Since many people today carry a smartphone at practically all times, an obvious solution to the problems of fitting processing power and display is to transmit the monitored data to the smartphone and use the processing power and display of the smartphone. Although it in this way would be possible to eliminate a display and reduce the processing power, using existing means of wireless communication requires relatively much power and space consuming electronics, all adding to volume and costs of the device.

2015/055592 on which the two-part form of claim 1 is based discloses a cap assembly comprising a needle assembly and a cartridge arranged in the interior thereof. WO 2015/140262 discloses a drug delivery device with cap induced needle movement.

Having regard to the above, it is a general object of the present invention to provide a durable (or semi-disposable) drug delivery device and system as well as components therefore which cost-effectively and reliably allows a high degree of user friendliness and convenience during both cartridge change and daily use.

It is a further object of the present invention to provide such a drug delivery device, system and components therefore which cost-effectively and reliably allows detection of dose data related to use of a drug delivery device.

### DISCLOSURE OF THE INVENTION

In the disclosure of the present invention, embodiments and aspects will be described which will address one or more of the above objects or which will address objects apparent from the below disclosure as well as from the description of exemplary embodiments.

Thus, in a first aspect of the invention a cap assembly as defined in claim 1 is provided.

By this arrangement a cap assembly is provided which cost-effectively and reliably allows a high degree of user friendliness and convenience during both cartridge change and daily use. The reservoir allows an amount of drug to be expelled into the sealed chamber via the hollow needle.

The cap assembly may be provided in combination with a drug delivery main unit, the main unit comprising, a housing, a piston rod, and a dose setting and expelling mechanism adapted to move the piston rod distally corresponding to a set dose. The cartridge and the drug delivery main unit are adapted to be releasably connected to each other, the piston rod thereby being arranged to engage and move the cartridge piston distally to expel an amount of drug from the cartridge via the hollow needle, the cap being releasably mounted relative to the cartridge and the thereto attached needle assembly. The cartridge may be arranged to be moved distally into engagement with the needle assembly corresponding to the operational state when the cap assembly in the initial state and the drug delivery main unit are connected to each other, i.e. the cartridge and the needle assembly engages each other "automatically" as the cap assembly in the initial state and the drug delivery main unit are connected to each other.

Such a concept may be described as "semi-disposable" primarily referring to the cartridge-containing cap assembly being disposable, however, the term semi-disposable may also be used to characterize the drug delivery main unit comprising the setting and expelling mechanism. More specifically, the main unit comprising the dose setting and expelling mechanism may be intended for use only with a limited number of cap assemblies. For example, the user may be provided with a box containing a number of cap assemblies and a single main unit which is intended to be disposed after having been used with the given number of cap assemblies in the box. Alternatively, the main unit may be a "durable" device intended to be used in combination with a non-specified number of cap assemblies over a longer period of time, e.g. 1-4 years.

In a specific embodiment the cap assembly comprises a cartridge unit, the cartridge unit comprising the above-described cartridge, cartridge unit first coupling means, cartridge unit second coupling means, and cartridge unit guide means. The cap further comprises cap guide means adapted to engage the cartridge unit guide means to provide guided axial movement there between. The needle assembly comprises needle coupling means adapted to engage the cartridge unit first coupling means corresponding to the operational state in which the proximal needle end has penetrated the cartridge seal. The cartridge unit can be moved axially from (i) an initial proximal position in which the proximal needle end is arranged distally of the cartridge seal, to (ii) a distal operational position in which the proximal needle end has penetrated the cartridge seal and the needle coupling means has engaged the cartridge unit first coupling means. In such an arrangement the cartridge may be arranged axially fixed in the cartridge unit, axial movement relative to the cap thereby taking place by movement of the cartridge unit.

The cartridge unit may comprise a generally tubular cartridge holder in which the cartridge is arranged. The described coupling and guide means may be formed as part of the cartridge holder. Alternatively, the cartridge unit first coupling means may be a separate member attached to the cartridge, e.g. as in a Penfill®, and subsequently inserted into the cartridge holder.

The needle assembly may be arranged axially moveable relative to the cap between an initial position in which the distal needle end is arranged proximally of the reservoir, and an operational position in which the distal needle end is arranged in the reservoir, wherein the needle assembly is moved from the initial position to the operational position when the cartridge unit is moved from the initial position to the operational position. Alternatively, the needle assembly may have an initial axial position in which the distal needle end is arranged in the reservoir.

In a specific embodiment the cap assembly comprising a cartridge unit may be provided in combination with a drug delivery main unit comprising a housing, a piston, a dose setting and expelling mechanism adapted to move the piston rod distally corresponding to a set dose, as well as main unit coupling means. The cap assembly and the drug delivery main unit are adapted to be releasably connected to each other, the piston rod being arranged to engage and move distally the cartridge piston to thereby expel an amount of drug from the cartridge via the hollow needle when operated by a user, wherein the main unit coupling means is adapted to releasably engage the cartridge unit second coupling means, e.g. formed as part of a cartridge holder.

The cartridge unit may be arranged to be moved distally into the distal operational position in which the proximal needle end has penetrated the cartridge seal and the needle coupling means has engaged the cartridge unit first coupling means when the cap assembly is connected to the drug delivery main unit.

The cartridge unit may further comprise third coupling means, and the cap may be provided with cap coupling means adapted to releasably engage the cartridge unit third coupling means. Such coupling means could also be used to keep the cartridge unit seated in the cap prior to mounting on a drug delivery main unit. Alternatively or in addition, coupling means may be provided between the cap and the drug delivery main unit.

As an alternative to using a cartridge holder to hold and position the cartridge in the cap, the cap assembly may be provided with support means holding and positioning the cartridge in the cap assembly, this allowing the cartridge to e.g. be inserted into a front loaded cartridge holder formed as part of the drug delivery main unit, the support means being arranged to brake away or to be pushed away when the distal portion of the drug delivery main unit is inserted into the cap.

In the above-described combinations of a cap assembly, comprising a cartridge or a cartridge unit, with a drug delivery main unit the piston rod may be arranged in a proximal-most initial position, e.g. pushed proximally by a user during a cartridge exchange procedure. The piston in a fully filled cartridge axially may be arranged within an initial pre-defined positional range, and the cartridge may be axially moveable relative to the cap from an initial to an operational position. In such an arrangement the cap assembly with the cartridge in the initial positon can be connected to the drug delivery main unit from (i) an initial axial position in which the piston rod engages the cartridge piston, via (ii) an intermediate axial position in which the cartridge has been moved to a distal-most position in the cap and into engagement with the needle assembly, the proximal needle end thereby penetrating the cartridge seal, and to (iii) an operational axial position in which the cartridge piston has been moved distally in the cartridge to thereby expel an amount of drug into the reservoir via the hollow needle. Alternatively the reservoir may be filled by manually expelling a small amount of drug into the reservoir, e.g. 2 IU of insulin.

In this way the reservoir can be filled with an amount of the drug contained in the cartridge without the user having to perform additional user steps during cartridge exchange. As the needle distal end is positioned in the reservoir during when the cap is mounted, the fluid drug in the reservoir may help prevent clocking of the needle. Additionally, when the reservoir is initially provided with a bacteriostatic substance or the fluid drug in the cartridge comprises a bacteriostatic preservative, the bacteriostatic fluid in the reservoir would provide that the distal needle end could be kept sterile before first use or cleaned during the time the needle distal end is parked in the reservoir, e.g. in case the user decides to re-use the needle.

The above-described drug delivery main units may be provided with dose logging circuitry adapted to determine variation of a parameter in the cartridge or in the drug delivery main unit being indicative of the size of a set or an expelled amount of drug, e.g. an amount of rotation of a component in the dose setting and expelling mechanism.

In a second aspect of the invention a cap assembly is provided, the cap assembly being supplied to the user in an initial state and comprises a cartridge filled with drug and defining a general axis, a hollow needle having a distal skin-piercing needle end and a proximal end in fluid communication with the cartridge, and a cap comprising a reservoir adapted to receive the needle distal end, the needle distal end in the initial state being received in the reservoir which is adapted to receive drug from the cartridge via the hollow needle.

The above-described cap assembly is intended for use in combination with a drug delivery main unit comprising a piston rod, and an expelling mechanism adapted to move the piston rod distally corresponding to a set or pre-set dose. The cartridge and the drug delivery main unit are adapted to be connected to each other, the piston rod being arranged to engage and move the cartridge piston distally to thereby expel an amount of drug from the cartridge via the hollow needle, the cap being releasably mounted relative to the cartridge to expose the distal skin-piercing needle end. The piston rod is or can be arranged in a proximal-most initial position, and the piston is, in a fully filled cartridge, in the initial state axially arranged within an initial pre-defined positional range. The cap assembly with the cartridge in the initial state is adapted to be connected to the drug delivery main unit from (i) an initial axial position in which the piston rod engages the cartridge piston, to (ii) an operational axial position in which the cartridge has been connected to the drug delivery main unit and the cartridge piston has been moved distally in the cartridge to thereby expel an amount of drug into the reservoir via the hollow needle.

As appears, by the above arrangement a simplified cap assembly is provided allowing a pre-connected needle to be used to fill a reservoir with drug from a cartridge.

The cap assembly may be provided with a cartridge unit as describe above. The pre-mounted needle may be in the form of a pre-mounted traditional needle assembly, this allowing the needle to be replaced, or the needle may be formed integrally with the cartridge unit or the cartridge, e.g. as in a prefilled syringe.

In a further aspect of the invention a drug delivery device (or assembly) comprising a first unit and a second unit adapted to be releasably connected to each other is provided. The first unit comprises a housing, a piston rod defining a general axis, a dose setting and expelling mechanism adapted to move the piston rod distally corresponding to a set dose, and first coupling means. The second unit comprises a drug-filled cartridge, the cartridge comprising an axially moveable piston and a distal portion with a needle penetratable seal. The second unit further comprises a needle assembly comprising a hollow needle having a distal skin-piercing needle end and a proximal seal-piercing needle end, a sealed chamber with a needle penetratable barrier adapted to receive the distal needle end, and second coupling means adapted to engage the first coupling means to thereby releasably connect the first and second units with the piston rod in engagement with the cartridge piston. The needle assembly and the cartridge are arranged axially moveable relative to each other between an initial state in which the proximal needle end is arranged distally of the cartridge seal, and an operational state in which the proximal needle end has penetrated the seal. When it is defined that the sealed chamber is provided with a needle penetratable barrier adapted to receive the distal needle end, this wording also covers embodiments in which the second unit initially is supplied to the user with the distal needle end arranged in the sealed chamber.

By the above arrangement a drug delivery device (or assembly) is provided which cost-effectively and reliably allows a high degree of user friendliness and convenience during both cartridge change and daily use. The sealed chamber allows an amount of drug to be expelled into the sealed chamber via the hollow needle.

Such an assembly may be described as "semi-disposable" primarily referring to the cartridge-containing second unit of the device assembly being disposable, however, the term semi-disposable may also be used to characterize the first unit comprising the setting and expelling mechanism (the main unit). More specifically, the main unit comprising the dose setting and expelling mechanism may be intended for use only with a limited number of cartridge units. For example, the user may be provided with a box containing a number of cartridge units and a single main unit which is intended to be disposed after having been used with given number of cartridge units in the box. Alternatively, the main unit may be a "durable" device intended to be used in combination with a non-specified number of cartridge units over a longer period of time, e.g. 1-4 years.

In an exemplary embodiment the second unit further comprises a releasably attached cap at least partially enclosing the cartridge, the cap comprising the sealed chamber. When the first and second units are connected to each other and the needle assembly and the cartridge are in the operational state, then the distal needle end with the cap in an attached state is arranged in the chamber penetrating the barrier, and the distal needle end with the cap in a detached state is free to be inserted through the skin of a user. The sealed chamber may be adapted to receive drug from the cartridge via the hollow needle. The sealed chamber may be vented to the interior of the cap.

In a specific embodiment the piston rod can be arranged in a proximal-most initial position, and the piston in a fully filled cartridge axially is arranged within an initial pre-defined positional range. The needle assembly comprises a needle hub adapted to be coupled, directly or indirectly, to the cartridge distal portion, and the cartridge is axially moveable relative to the cap from an initial to an operational position. In such an arrangement the second unit with the cartridge in the initial positon can be connected to the first unit from (i) an initial axial position in which the piston rod engages the cartridge piston, via (ii) an intermediate axial position in which the cartridge has been moved to a distal-most position in the cap and into coupling engagement with the needle hub, the proximal needle end thereby penetrating the cartridge seal, and to (iii) an operational axial position in which the cartridge piston has been moved distally in the cartridge to thereby expel an amount of drug into the chamber via the hollow needle.

The needle assembly may be arranged axially non-movable relative to the cap and in a distal-most position when the second unit with the cartridge in the initial positon is connected to the first unit.

In an exemplary embodiment the second unit comprises a cartridge holder portion in which the cartridge is arranged axially fixed, the cartridge holder portion being arranged axially movable in the cap from an initial to an operational position, wherein the cartridge holder portion comprises the second coupling means.

The first unit may be provided with electronic circuitry adapted to determine variation of a parameter in the drug delivery device being indicative of the size of a set or an expelled amount of drug. The detected parameter may be rotation of a component in the dose setting and expelling mechanism, e.g. rotation of a transmission member upon which a drive spring is acting, the drive spring being strained during dose setting and released during dose expelling.

In a second aspect of the invention a cartridge unit is provided, comprising a cartridge holder, a drug-filled cartridge arranged in the cartridge holder, the cartridge comprising an axially moveable piston and a distal portion with a needle penetratable seal, a needle assembly comprising a hollow needle having a distal skin-piercing needle end and a proximal seal-piercing needle end, and a releasable attached cap comprising a sealed chamber with a needle penetratable barrier, the cap at least partially housing the cartridge and cartridge holder, the sealed chamber being adapted to receive an amount of fluid drug from the cartridge. The cartridge is arranged to be axially moveable relative to the cap from an initial position in which there is no fluid communication between the hollow needle and the cartridge, and to an operational position in which the needle proximal end has penetrated the cartridge seal.

The needle assembly may comprise a needle hub adapted to be coupled, directly or indirectly, to the cartridge distal portion, wherein the cartridge in the operational position is in coupling engagement with the needle hub. With the cartridge in the initial position, the hollow needle may be arranged in flow communication with the sealed chamber.

The cartridge may be arranged axially fixed in the cartridge holder, and the cartridge holder may be arranged axially movable in the cap from an initial to an operational position.

The cartridge holder may comprise coupling means allowing the cartridge holder to be connected to a drug delivery device comprising a piston rod adapted to engage the cartridge piston.

In a yet further aspect of the invention a drug delivery device (or assembly) comprising a first unit and a second unit adapted to be releasably connected to each other is provided. The first unit comprises a housing, a piston rod defining a general axis, a dose setting and expelling mechanism adapted to move the piston rod distally corresponding to a set dose, and first coupling means. The second unit comprises a drug-filled cartridge, the cartridge comprising an axially moveable piston and a distal portion with a needle penetratable seal. The second unit further comprises a needle assembly comprising a hollow needle having a distal skin-piercing needle end and a proximal seal-piercing needle end, and second coupling means adapted to engage the first coupling means to thereby releasably connect the first and second units with the piston rod in engagement with the cartridge piston. The needle assembly and the cartridge are arranged axially moveable relative to each other between an initial state in which the proximal needle end is arranged distally of the cartridge seal, and an operational state in which the proximal needle end has penetrated the seal.

As used herein, the term "drug" is meant to encompass any flowable medicine formulation capable of being passed through a delivery means such as a cannula or hollow needle in a controlled manner, such as a liquid, solution, gel or fine suspension, and containing one or more drug agents. The drug may be a single drug compound or a premixed or co-formulated multiple drug compounds drug agent from a single reservoir. Representative drugs include pharmaceuticals such as peptides (e.g. insulins, insulin containing drugs, GLP-1 containing drugs as well as derivatives thereof), proteins, and hormones, biologically derived or active agents, hormonal and gene based agents, nutritional formulas and other substances in both solid (dispensed) or liquid form. In the description of the exemplary embodiments reference will be made to the use of insulin and GLP-1 containing drugs, this including analogues thereof as well as combinations with one or more other drugs.

### BRIEF DESCRIPTION OF THE DRAWINGS

In the following the invention will be further described with reference to the drawings, wherein
fig. 1 shows a drug delivery device main unit with a mounted cartridge unit,
fig. 2 shows the drug delivery device main unit of fig. 1 detached from the cartridge unit,
fig. 3 shows a cartridge unit in a partial see-through representation, the cartridge being in a proximal position,
fig. 4A shows the cartridge unit of fig. 3 in a partial cross-sectional representation, the cartridge being in a distal position,
fig. 4B shows the cartridge unit of fig. 4A in a cross-sectional representation,
fig. 5 shows the drug delivery device main unit of fig. 1 in a cross-sectional view,
figs. 6A-6E show in cross-section and in different states the cartridge unit being mounted on the drug delivery device main unit, and
fig. 7 shows in cross-section the cartridge unit fully mounted on the drug delivery device main unit with the cap member being removed.

In the figures like structures are mainly identified by like reference numerals.

### DESCRIPTION OF EXEMPLARY EMBODIMENTS

When in the following terms such as "upper" and "lower", "right" and "left", "horizontal" and "vertical" or similar relative expressions are used, these only refer to the appended figures and not necessarily to an actual situation of use. The shown figures are schematic representations for which reason the configuration of the different structures as well as their relative dimensions are intended to serve illustrative purposes only. When the term member or element is used for a given component it generally indicates that in the described embodiment the component is a unitary component, however, the same member or element may alternatively comprise a number of sub-components just as two or more of the described components could be provided as unitary components, e.g. manufactured as a single injection moulded part. When it is defined that members are mounted axially free to each other it generally indicates that they can be moved relative to each other, e.g. between defined stop positions whereas when it is defined that members are mounted rotationally free to each other it generally indicates that they can be rotated relative to each other either freely or between defined stop positions. The terms "assembly" and "subassembly" do not imply that the described components necessarily can be assembled to provide a unitary or functional assembly or subassembly during a given assembly procedure but is merely used to describe components grouped together as being functionally more closely related.

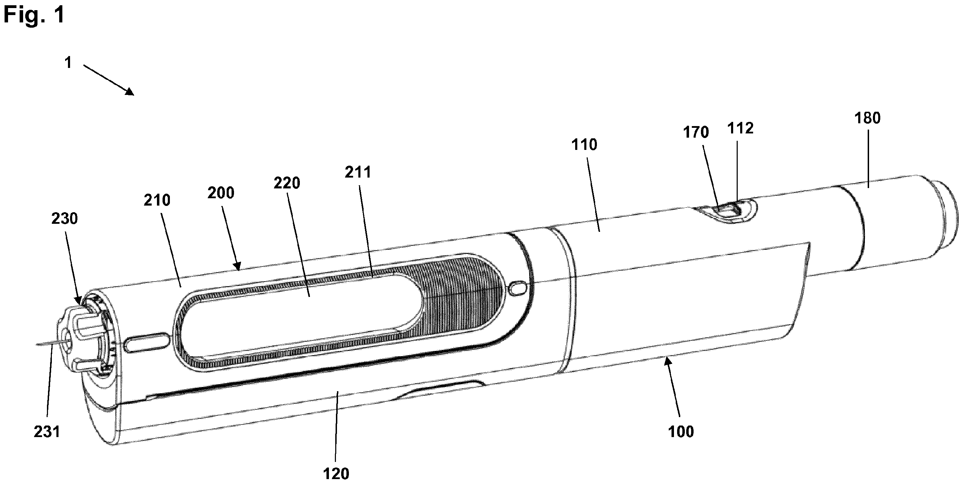

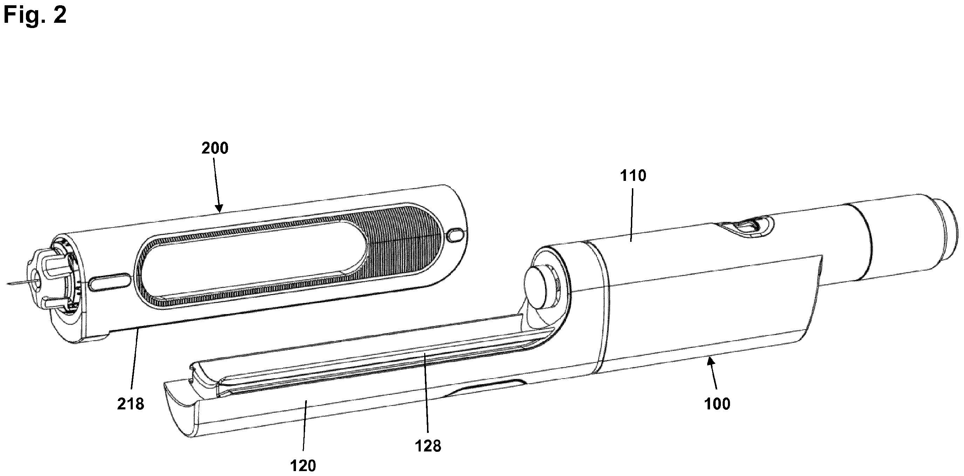

Referring to figs. 1 and 2 a pen-formed drug delivery device 1 will be described. More specifically, the pen device 1 is in the form of an assembly comprising a drug delivery main unit 100 which in the shown operational state in fig. 1 is releasably coupled to a cartridge unit 200. Fig. 2 shows the cartridge unit removed from the main unit.

The cartridge unit 200 comprises a generally tubular cartridge holder 210 in which a drug-filled transparent cartridge 220 is arranged, the cartridge comprising a proximally facing and axially moveable piston and a distal portion with a needle penetratable septum. The cartridge may for example contain an insulin, a GLP-1 or a growth hormone formulation. A pair of opposed openings 211 formed in the cartridge holder allows the cartridge and its content to be inspected by a user. The cartridge unit further comprises a needle assembly 230 comprising a hollow needle having a distal skin-piercing needle portion 231 and a proximal septum-piercing needle portion 232 which in the shown state is arranged in fluid communication with the cartridge interior.

The drug delivery main unit 100 comprises a proximal body portion 110 and a distally extending guide portion 120. A dose setting and expelling mechanism is arranged in the proximal body portion and comprises a piston rod adapted to engage the cartridge piston of a mounted cartridge unit to thereby expel a dose of drug via the hollow needle as it is moved distally. A proximal rotatable dose setting member 180 serves to manually set a desired dose of drug shown in a display window 112 and which can then be expelled when the release button 190 is actuated. The window is in the form of an opening in the housing allowing a portion of a helically rotatable indicator member (scale drum) 170 to be observed. Depending on the type of expelling mechanism embodied in the drug delivery device, the expelling mechanism may comprise a spring as in the shown embodiment which is strained during dose setting and then released to drive the piston rod when the release button is actuated. Alternatively the expelling mechanism may be fully manual in which case the dose member and the actuation button move proximally during dose setting corresponding to the set dose size, and then is moved distally by the user to expel the set dose.

The distally extending guide portion 120 comprises guide means 128 adapted to engage corresponding guide means 218 arranged on the cartridge holder, the guide means allowing the cartridge unit to be mounted in an axial direction in sliding engagement with the guide portion of the device main unit. Fig. 1 shows the cartridge unit 200 being fully mounted in a proximal-most position relative to the main unit 100, the two units being releasably coupled to each other by corresponding releasable coupling means. The coupling means may be formed as part of the guide means 128, 218. Alternatively the coupling means may be arranged on the main unit body portion and the corresponding portion on the cartridge unit.

The drug delivery device further comprises a cap member (see below) adapted to cover the cartridge unit (and in the shown embodiment also the guide portion) when mounted. As will be described in greater detail below, the cap in the shown embodiment comprises a reservoir in the form of a sealed chamber adapted to receive an amount of preservative-containing drug from the cartridge.

The shown drug delivery device 1 may be provided with electronic circuitry comprising sensor means adapted to detect a property related to the size of a set and/or expelled dose amount of drug, e.g. the amount of rotation of an indicator member during dose expelling. The electronic circuitry may be provided with display means allowing determined dose sizes to be displayed and/or communication means allowing dose related data to be transmitted to an external device, e.g. wirelessly to a smartphone for subsequent analysis and display.

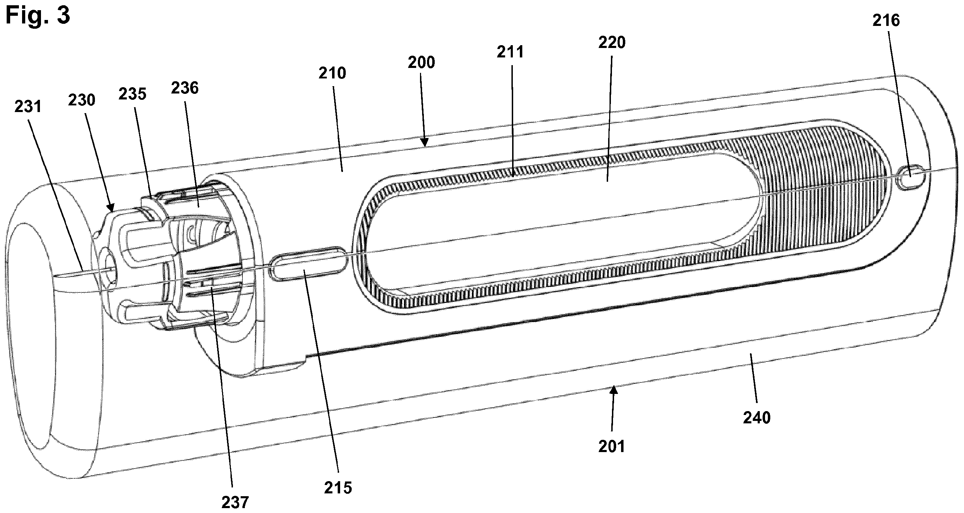

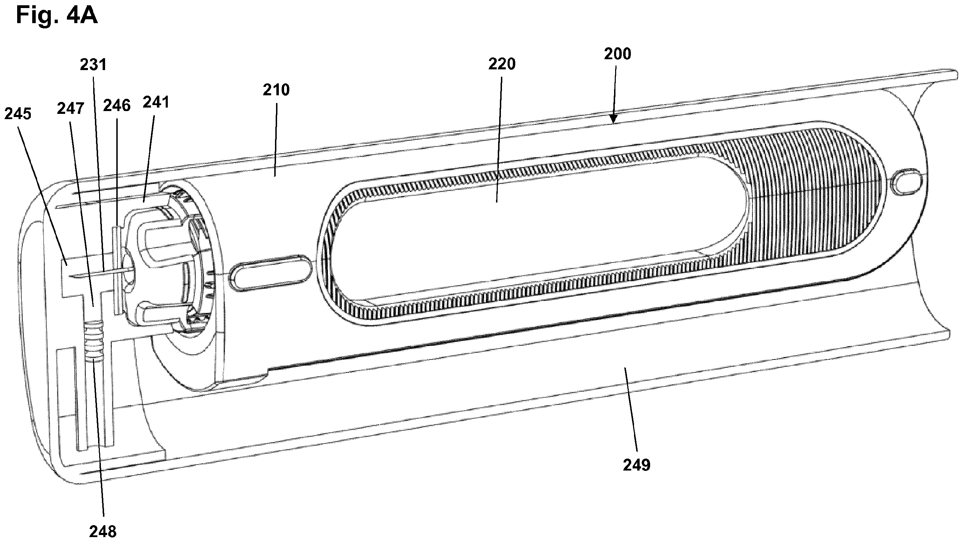

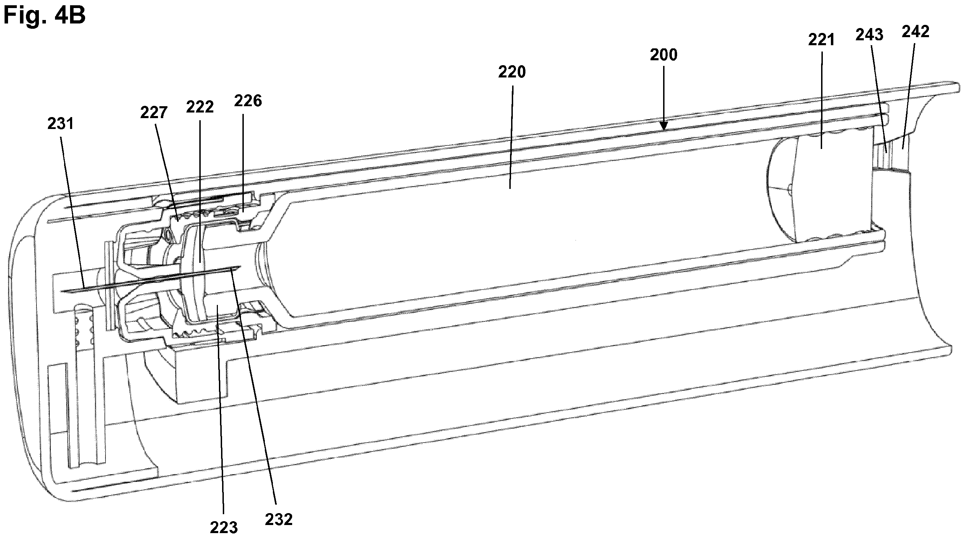

With reference to figs. 3, 4A and 4B the cartridge unit 200 will be described in greater detail.

Referring first to fig. 3 the cartridge holder 210 and the needle assembly 230 is shown in their initial before-use position inside cap member 240, the latter represented by an exterior outline but without any interior details. In the following the combined cap and cartridge unit assembly will be denoted cap assembly 201. A drug-filled cartridge 220 with an axially displaceable piston 221 and a distal needle-penetratable septum 222 is arranged axially locked inside the cartridge holder. The cartridge comprises a reduced-diameter distal portion 223 providing a circumferential coupling space between the cartridge distal portion and the cartridge holder distal portion. The cartridge holder is coupled non-rotationally but axially moveable to the cap, and is moveable in a distal direction between an initial proximal position as shown in fig. 3 and an operational distal position as shown in figs. 4A and 4B. In the shown embodiment the cartridge holder is coupled to the cap by means of two pairs of opposed protrusions 215, 216 engaging corresponding slots 242 in the cap, the coupling (or guide) subsequently allowing the cap to be removed from and remounted on the cartridge holder. Between the cartridge holder and the cap a releasable axial lock is provided to properly secure the cartridge holder in the initial position prior to use, e.g. by means of protrusions 243 in the guiding slots adapted to cooperate with the protrusions 216 on the cartridge holder, the lock being designed to be overcome by an axial force applied to the cartridge holder.

Along the length of the cartridge holder a longitudinal space 249 is formed between the cartridge holder and the cap inner surface, the space being adapted to receive and accommodate the above-described main unit guide portion.

The needle assembly 230 comprises a hub member 235 in which the hollow needle with its distal skin-piercing needle portion 231 and proximal septum-piercing needle portion 232 is mounted, the hub member being releasably mounted in a "parked" position by gripping means 241 formed in the cap member, the parked position corresponding to both an initial before-use position and the position during use with the cap mounted. The hub member comprises a proximally facing circumferential skirt portion 236 adapted to be received in the coupling space (as shown in figs. 4A and 4B), the skirt portion comprising coupling means 237 adapted to engage corresponding coupling means on the cartridge and/or cartridge holder. In the shown embodiment the coupling means is in the form of a threaded outer surface 227 formed on a generally tubular coupling member 226 mounted on the cartridge distal portion 223, and a number of flexible gripping ribs 237 formed on the skirt inner surface, the coupling arrangement allowing the skirt portion to axially engage the threaded surface, yet allows the needle assembly to be removed by rotation if deemed necessary, e.g. in order to allow a damaged needle to be replaced. Alternatively the coupling may be non-releasable.

The cap is further provided with a sealed chamber 245 comprising first and second openings. The first opening is sealed with a needle-penetratable septum 246 and the second opening is in the form of a tubular bore 247 in which a plug 248 formed from a flexible material is arranged, the plug serving as a seal as well as a pressure/overflow relief valve in communication with the interior of the cap. With the needle assembly 230 in its parked position the distal needle portion 231 is arranged in the sealed chamber 245 penetrating the septum 246. In the initial state the shown sealed chamber is empty, however, as will be explained below the chamber is adapted to receive and hold an amount of preservative-containing drug from the cartridge, the preservatives ensuring a sterile or near-sterile environment for the needle distal portion in the parked position, this allowing the needle to be re-used corresponding to the intended use-time for the cartridge unit. Indeed, prior to use the cartridge unit will have to be designed and packaged in such a way that sterility of both the proximal and distal ends of the needle is safeguarded.

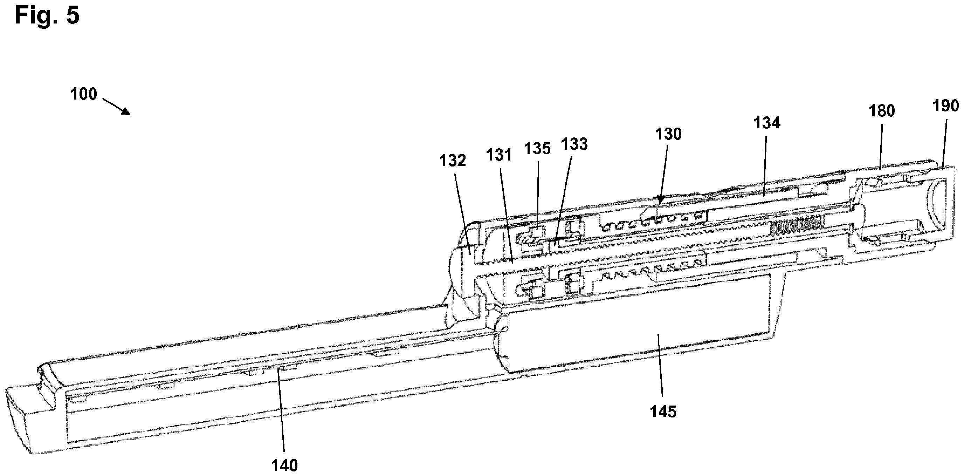

Fig. 5 shows in cross-section a schematic representation of the drug delivery main unit 100 comprising a dose setting and expelling mechanism 130, electronic circuitry 140 arranged in the guide portion as well as an energy source, e.g. a replaceable or non-replaceable AAA or AAAA "battery" 145 as shown in case the main unit is of the durable type. The dose setting and expelling mechanism is only shown schematically comprising a threaded piston rod 131 having a distal piston-engaging foot portion 132, a driver 133 for rotating the piston rod, a scale drum 134, a drive spring (not shown), a dose setting member 180, a release member 190 and a coupling mechanism 135 allowing the piston rod to be returned to an initial proximal position during exchange of a cartridge unit. Alternatively the foot portion may be in the form of a piston washer connected to the piston rod to allow the washer to wobble. The dose setting and expelling mechanism may be of any suitable design, e.g. utilizing the design and concepts described in EP 17151586.9. Correspondingly, the electronic circuitry and the associated sensor means may be of any suitable design allowing a relevant dose related property to be determined, e.g. a rotary sensor assembly of the type described in EP 17151586.9 or a magnet-based system as described in WO 2014/161952.

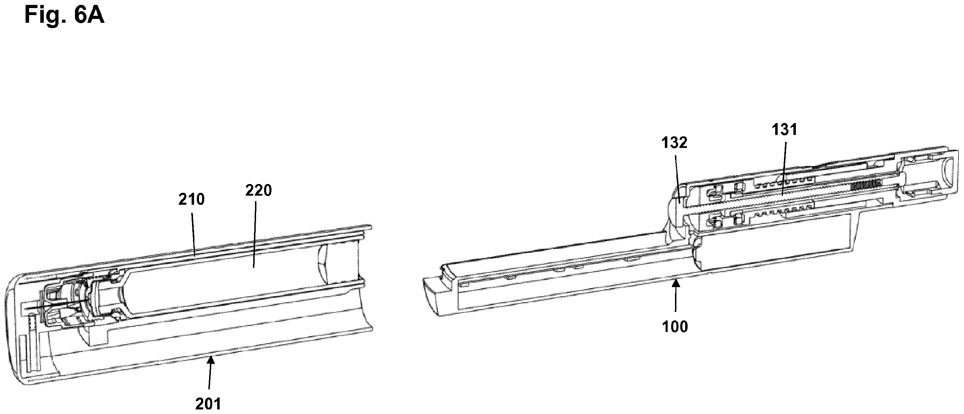

With reference to figs. 6A-6E the mounting of a cap assembly and thereby a cartridge unit on the drug delivery main unit will be described. The cap assembly 201 and the cartridge unit 200 correspond to the cap and cartridge units described with reference to figs. 3, 4A and 4B, and the main unit 100 corresponds to the main unit described with reference to fig. 5.

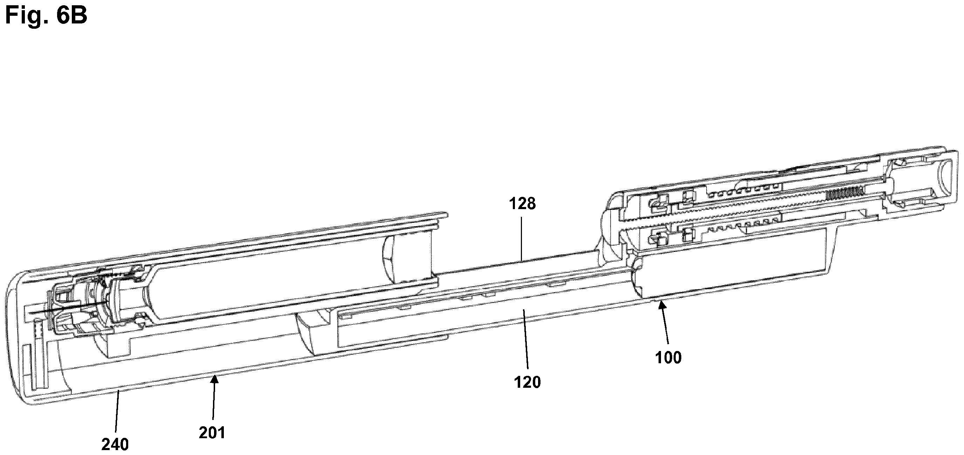

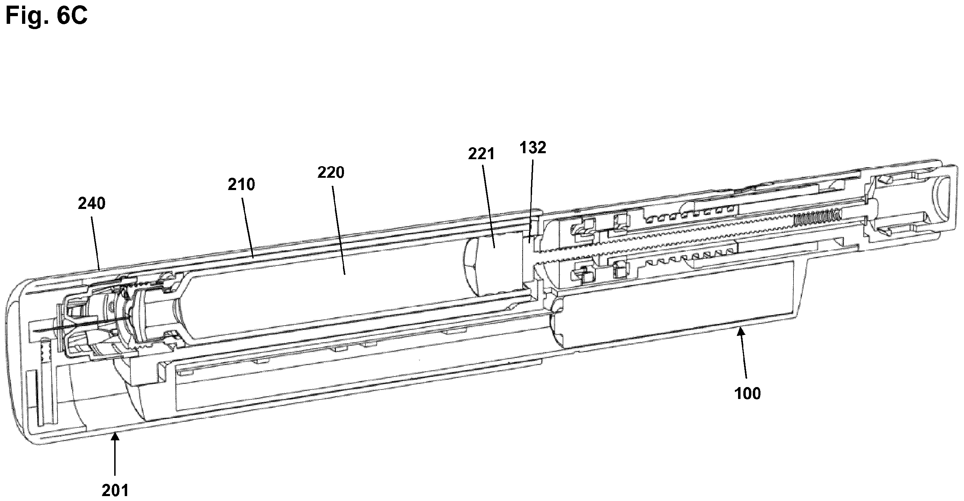

Fig. 6A shows the cap assembly 201 and main unit 100 separated from each other, the piston rod 131 in the main unit being positioned in its proximal-most position. The cap assembly 201 is in an initial state with the cartridge holder 210 in a proximal position and the drug-filled cartridge 220 thus not yet in flow communication with the hollow needle 232. In fig. 6B the guide and coupling means 128 on the main unit distal guide portion has been mated with the corresponding guide and coupling means 218 on the cartridge holder, this allowing the guide portion 120 to be introduced into the receiving space 249 in the cap. In fig. 6C the guide portion 120 has been inserted further into the cap 240 to the position in which the piston rod distal end 132 just engages the free end of the cartridge piston 221. At this point the user will feel a resistance to further insertion of the guide portion due to the axial lock between the cartridge holder 210 and the cap member 240.

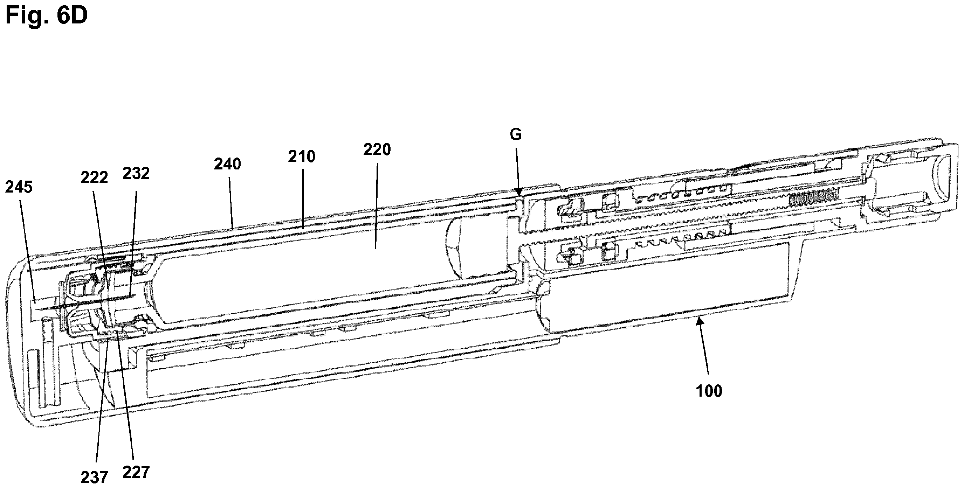

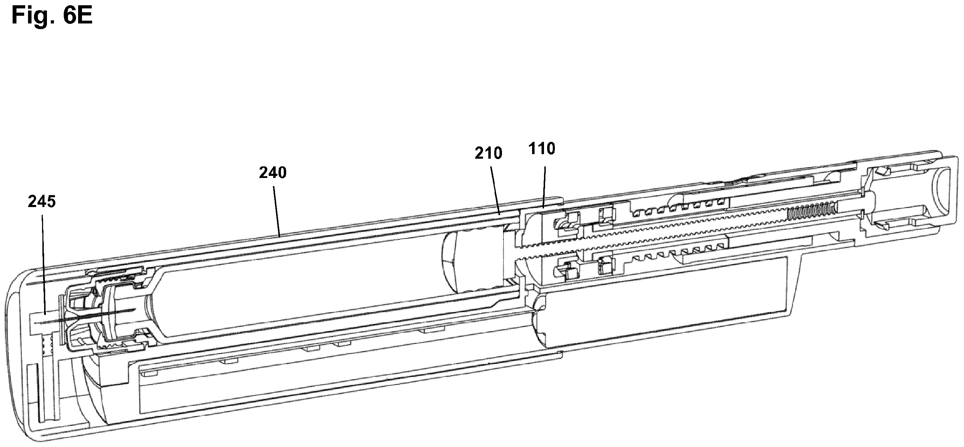

When the resistance is overcome and the axial lock released the engagement with the piston rod 131 will prevent the sealed drug-filled cartridge 220 and thus the cartridge holder 210 from being moved further proximally relative to the main unit 100, this providing that the cap 240 with the cap-mounted needle assembly 230 is moved proximally, the skirt portion 235 thereby being inserted in the circumferential coupling space. As a result the proximal needle portion 232 penetrates the cartridge septum 222 and the skirt portion coupling means 237 engages the corresponding coupling means 227 on the cartridge as shown in fig. 6D, a flow communication between the cartridge interior and the cap sealed chamber 245 thereby being established. As the cartridge holder 210 has been moved to its distal-most position in the cap member 240 further advancement of the guide portion 120 into the cap results in the cartridge piston 221 being moved distally in the cartridge until the cartridge holder 210 engages the main unit housing 110 as shown in fig. 6E, whereby an amount of fluid drug is transferred to the cap sealed chamber 245, the amount corresponding to the distance G between the cartridge holder and the main unit housing shown in fig. 6D. Indeed, for this to happen it is necessary that the cartridge piston 221 initially is positioned within a pre-defined axial positional range, the range ensuring that the sealed chamber 245 as a minimum is filled with the minimally required amount of preservative-containing drug, or as a maximum with an amount of drug resulting in an overflow of an acceptable small amount of drug out into the cap interior. A fluid trap for this purpose may be provided in the cap.

Although in the above description the distal movement of the cartridge holder and the transfer of drug to the sealed chamber are described as taking part in sequence, a certain overlap in time between the two events may be the case.

When the cartridge holder engages the main unit, the two units are releasably locked to each other, e.g. by means of a snap coupling. At the same time the piston rod coupling is actuated from a resetting state in which it can be moved to its proximal-most position, to an operational state in which it is coupled to the expelling mechanism for subsequent movement in the distal direction during expelling of a dose of drug from the cartridge. Correspondingly, when a used cartridge unit is released from the main unit the piston rod coupling is actuated to its resetting state allowing the piston rod to be moved proximally, either by hand prior to attaching a new cartridge unit or simply by allowing the new cartridge unit to move the piston rod proximally to its proximally-most position. Indeed, the resistance to axial movement of the piston rod should be sufficiently low to prevent that the cartridge is released from its locked position in the cap. To further simplify removal of a used cartridge unit from the main unit, the cap may be designed to allow the snap lock to be released without the cap member having to be removed.

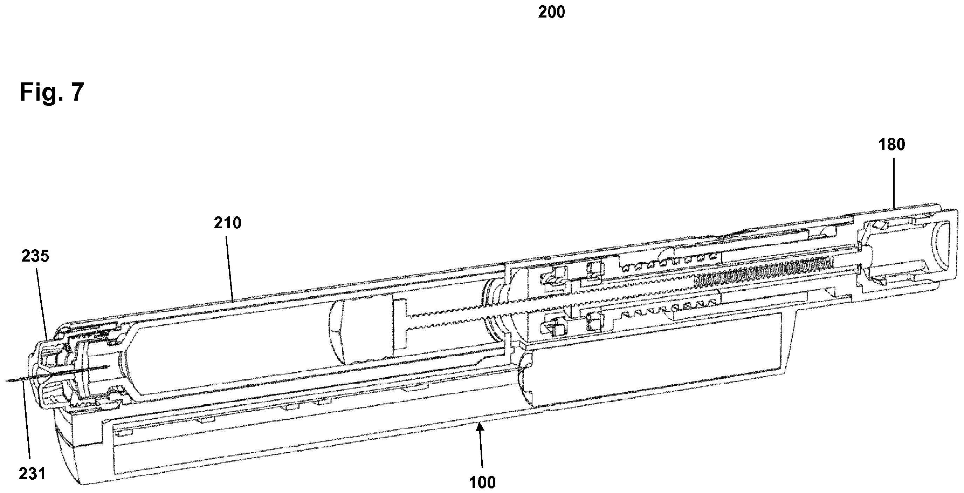

As appears, by the above arrangement a semi-disposable drug delivery system is provided which offers a very high degree of simplicity and user-friendliness, as a new cartridge unit can be mounted and primed and be ready for use virtually in a few seconds. Correspondingly, when a dose has been set the user can remove the cap and immediately use the device to dispense the set dose without having to bother with the mounting of a new needle assembly, see fig. 7. Indeed, the dose can also be set after the cap has been removed.

Further, in the shown embodiment a relatively large replaceable battery has been incorporated, this allowing "high-power" wireless communication to be implemented, e.g. providing safe and reliable communication with e.g. a smartphone carried by the user without having to position the two devices in the vicinity of each other.

In the above description of exemplary embodiments, the different structures and means providing the described functionality for the different components have been described to a degree to which the concept of the present invention will be apparent to the skilled reader. The detailed construction and specification for the different components are considered the object of a normal design procedure performed by the skilled person along the lines set out in the present specification.

## Claims

1. A cap assembly (201) adapted to be attached to a drug delivery main unit (100), comprising:
- a cartridge (220) filled with a drug and defining a general axis, the cartridge comprising an axially moveable piston (221) and a distal portion with a needle penetratable seal (222),
- a needle assembly (230) comprising a hollow needle having a distal skin-piercing needle end (231) and a proximal seal-piercing needle end (232), and
- a cap (240) accommodating the needle assembly and at least the distal portion of the cartridge,
wherein the cartridge (220) is arranged to be moved distally into engagement with the needle assembly (230) between an initial state in which the proximal needle end (232) is arranged distally of the cartridge seal (222), and an operational state in which the proximal needle end has penetrated the cartridge seal,
**characterized in that**
- the cap comprises a sealed chamber (245) with a needle penetratable barrier (246) adapted to receive the distal needle end (231), and
- the sealed chamber is adapted to receive drug from the cartridge via the hollow needle.

2. A cap assembly as in claim 1, in combination with a drug delivery main unit (100) comprising:
- a housing (110),
- a piston rod (131, 132), and
- a dose setting and expelling mechanism (130) adapted to move the piston rod distally corresponding to a set dose,
wherein the cartridge (220) and the drug delivery main unit (100) are adapted to be releasably connected to each other, the piston rod being arranged to engage and move the cartridge piston distally to thereby expel an amount of drug from the cartridge via the hollow needle, the cap being releasably mounted relative to the cartridge.

3. A cap assembly in combination with a drug delivery main unit as in claim 2, wherein the cartridge (220) is arranged to be moved distally into engagement with the needle assembly (230) corresponding to the operational state when the cap assembly in the initial state and the drug delivery main unit are connected to each other.

4. A cap assembly as in claim 1, further comprising a cartridge unit (200), the cartridge unit comprising:
- the cartridge (220),
- cartridge unit first coupling means (227),
- cartridge unit second coupling means (218), and
- cartridge unit guide means (215, 216),
the cap (240) further comprising cap guide means (242) adapted to engage the cartridge unit guide means to provide guided axial movement there between,
wherein the needle assembly (230) comprises needle coupling means (237) adapted to engage the cartridge unit first coupling means (227) corresponding to the operational state in which the proximal needle end has penetrated the cartridge seal, and
wherein the cartridge unit (200) can be moved axially from an initial proximal position in which the proximal needle end (232) is arranged distally of the cartridge seal (222), to a distal operational position in which the proximal needle end has penetrated the cartridge seal and the needle coupling means has engaged the cartridge unit first coupling means.

5. A cap assembly as in claim 4, wherein the needle assembly (230) has an initial axial position in which the distal needle end is arranged in the reservoir.

6. A cap assembly as in claim 4, wherein:
- the needle assembly (230) is arranged axially moveable relative to the cap between an initial position in which the distal needle end (231) is arranged proximally of the reservoir (245), and an operational position in which the distal needle end is arranged in the reservoir, and
- the needle assembly (230) is moved from the initial position to the operational position when the cartridge unit is moved from the initial position to the operational position.

7. A cap assembly as in any of claims 4-6, in combination with a drug delivery main unit (100) comprising:
- a housing (110),
- a piston rod (131, 132),
- a dose setting and expelling mechanism (130) adapted to move the piston rod distally corresponding to a set dose, and
- main unit coupling means (128),
wherein the cap assembly (201) and the drug delivery main unit (100) are adapted to be releasably connected to each other, the piston rod (131, 132) being arranged to engage and move distally the cartridge piston (221) to thereby expel an amount of drug from the cartridge via the hollow needle, and
wherein the main unit coupling means (128) is adapted to releasably engage the cartridge unit second coupling means (218).

8. A cap assembly in combination with a drug delivery main unit as in claim 7, wherein the cartridge unit (200) is arranged to be moved distally into the distal operational position in which the proximal needle end (232) has penetrated the cartridge seal (222) and the needle coupling means has engaged the cartridge unit first coupling means when the cap assembly is connected to the drug delivery main unit.

9. A cap assembly in combination with a drug delivery main unit as in claim 7 or 8, wherein the cartridge unit (200) further comprises third coupling means (216), and the cap further comprising cap coupling means (243) adapted to releasably engage the cartridge unit third coupling means.

10. A cap assembly (200) in combination with a drug delivery main unit (100) as in any of claims 2, 3, 7-9, wherein:
- the piston rod (131, 132) can be arranged in a proximal-most initial position,
- the piston (221) in a fully filled cartridge axially is arranged within an initial pre-defined positional range, and
- the cartridge (220) is axially moveable relative to the cap (240) from an initial to an operational position,
wherein the cap assembly (200) with the cartridge in the initial positon can be connected to the drug delivery main unit (100) from:
(i) an initial axial position in which the piston rod (131, 132) engages the cartridge piston (221), via
(ii) an intermediate axial position in which the cartridge (220) has been moved to a distal-most position in the cap (240) and into engagement with the needle assembly (235), the proximal needle end (232) thereby penetrating the cartridge seal (222), and to
(iii) an operational axial position in which the cartridge piston (221) has been moved distally in the cartridge to thereby expel an amount of drug into the reservoir (245) via the hollow needle (231, 232).

11. A cap assembly (200) in combination with a drug delivery main unit (100) as in any of claims 2, 3, 7-10, wherein the drug delivery main unit further comprises dose capture circuitry (140) adapted to determine variation of a parameter in the cap assembly or the drug delivery main unit being indicative of the size of a set and/or an expelled amount of drug.

12. A cap assembly (200) in combination with a drug delivery main unit (100) as in claim 11, wherein the detected parameter is rotation of a component in the dose setting and expelling mechanism (130).

13. A cap assembly (200) in combination with a drug delivery main unit (100) as in claim 11 or 12, further comprising wireless communication means allowing captured data to be transmitted to an external device.

14. A cap assembly (200) in combination with a drug delivery main unit (100) as in any of claims 2, 3, 7-13, wherein the sealed chamber (245) is vented (248) to the interior of the cap.

## Patentansprüche

1. Kappenanordnung (201), ausgebildet zur Anbringung an einer Medikamentenabgabe-Haupteinheit (100), umfassend:
- eine mit einem Medikament gefüllte und eine allgemeine Achse definierende Patrone (220), wobei die Patrone einen axial bewegbaren Kolben (221) und einen distalen Abschnitt mit einer von einer Nadel durchdringbaren Dichtung (222) umfasst,
- eine Nadelanordnung (230), die eine Hohlnadel mit einem distalen, die Haut durchstechenden Nadelende (231) und einem proximalen, die Dichtung durchstechenden Nadelende (232) umfasst, und
- eine Kappe (240), die die Nadelanordnung und zumindest den distalen Teil der Patrone aufnimmt,
wobei die Patrone (220) so angeordnet ist, dass sie distal in Eingriff mit der Nadelanordnung (230) zwischen einem Anfangszustand, in dem das proximale Nadelende (232) distal von der Patronendichtung (222) angeordnet ist, und einem Betriebszustand, in dem das proximale Nadelende die Patronendichtung durchdrungen hat, bewegt werden kann, **dadurch gekennzeichnet, dass**
- die Kappe eine abgedichtete Kammer (245) mit einer von einer Nadel durchdringbaren Barriere (246) umfasst, die zur Aufnahme des distalen Nadelendes (231) ausgebildet ist, und
- die abgedichtete Kammer so ausgebildet ist, dass sie das Medikament über die Hohlnadel aus der Patrone aufnehmen kann.

2. Kappenanordnung nach Anspruch 1 in Kombination mit einer Medikamentenabgabe-Haupteinheit (100), umfassend:
- ein Gehäuse (110),
- eine Kolbenstange (131, 132), und
- einen Dosiseinstellungs- und -ausstoßmechanismus (130), der zur Bewegung der Kolbenstange in distaler Richtung entsprechend einer eingestellten Dosis ausgebildet ist, wobei die Patrone (220) und die Medikamentenabgabe-Haupteinheit (100) zur lösbaren Verbindung miteinander ausgebildet sind, wobei die Kolbenstange derart angeordnet ist, dass sie in den Patronenkolben eingreift und diesen in distaler Richtung bewegt, um dadurch eine Medikamentenmenge aus der Patrone über die Hohlnadel auszustoßen, wobei die Kappe relativ zur Patrone lösbar angebracht ist.

3. Kappenanordnung in Kombination mit einer Medikamentenabgabe-Haupteinheit nach Anspruch 2, wobei die Patrone (220) angeordnet ist, dass sie distal in Eingriff mit der Nadelanordnung (230) entsprechend dem Betriebszustand bewegt werden kann, wenn die Kappenanordnung im Ausgangszustand und die Medikamentenabgabe-Haupteinheit miteinander verbunden sind.

4. Kappenanordnung nach Anspruch 1, ferner umfassend eine Patroneneinheit (200), wobei die Patroneneinheit umfasst:
- die Patrone (220),
- erste Kupplungsmittel der Patroneneinheit (227),
- zweite Kupplungsmittel der Patroneneinheit (218) und
- Patroneneinheit-Führungsmittel (215, 216),
die Kappe (240), ferner umfassend Kappenführungsmittel (242), die ausgebildet sind, um mit dem Führungsmittel der Patroneneinheit in Eingriff zu kommen, um eine geführte axiale Bewegung dazwischen zu ermöglichen,
wobei die Nadelanordnung (230) Nadelkupplungsmittel (237) umfasst, die geeignet sind, mit den ersten Kupplungsmitteln der Patroneneinheit (227) entsprechend dem Betriebszustand in Eingriff zu kommen, in dem das proximale Nadelende die Patronendichtung durchdrungen hat, und
wobei die Patroneneinheit (200) axial von einer anfänglichen proximalen Position, in der das proximale Nadelende (232) distal von der Patronendichtung (222) angeordnet ist, in eine distale Betriebsposition bewegt werden kann, in der das proximale Nadelende die Patronendichtung durchdrungen hat und die Nadelkupplungsmittel in die ersten Kupplungsmittel der Patroneneinheit eingegriffen haben.

5. Kappenanordnung nach Anspruch 4, wobei die Nadelanordnung (230) eine anfängliche axiale Position aufweist, in der das distale Nadelende im Reservoir angeordnet ist.

6. Kappenanordnung nach Anspruch 4, wobei:
- die Nadelanordnung (230) relativ zur Kappe zwischen einer Ausgangsposition, in der das distale Nadelende (231) proximal des Reservoirs (245) angeordnet ist, und einer Betriebsposition, in der das distale Nadelende in dem Reservoir angeordnet ist, axial beweglich angeordnet ist, und
- die Nadelbaugruppe (230) von der Ausgangsposition in die Betriebsposition bewegt wird, wenn die Patroneneinheit von der Ausgangsposition in die Betriebsposition bewegt wird.

7. Kappenanordnung nach einem der Ansprüche 4-6, in Kombination mit einer Medikamentenabgabe-Haupteinheit (100), umfassend:
- ein Gehäuse (110),
- eine Kolbenstange (131, 132),
- einen Dosiseinstellungs- und -ausstoßmechanismus (130), der ausgelegt ist, die Kolbenstange distal entsprechend einer eingestellten Dosis zu bewegen, und
- Kupplungsmittel der Haupteinheit (128),
wobei die Kappenanordnung (201) und die Medikamentenabgabe-Haupteinheit (100) zur lösbaren Verbindung miteinander ausgebildet sind, wobei die Kolbenstange (131, 132) angeordnet ist, um in den Patronenkolben (221) einzugreifen und diesen distal zu bewegen, um dadurch eine Medikamentenmenge aus der Patrone über die Hohlnadel auszustoßen, und
wobei das Kupplungsmittel der Haupteinheit (128) dazu ausgelegt ist, das zweite Kupplungsmittel der Patroneneinheit (218) lösbar in Eingriff zu bringen.

8. Kappenanordnung in Kombination mit einer Medikamentenabgabe-Haupteinheit nach Anspruch 7, wobei die Patroneneinheit (200) angeordnet ist, um distal in die distale Betriebsposition bewegt zu werden, in der das proximale Nadelende (232) die Patronendichtung (222) durchdrungen hat und die Nadelkupplungsmittel in die ersten Kupplungsmittel der Patroneneinheit eingegriffen haben, wenn die Kappenanordnung mit der Medikamentenabgabe-Haupteinheit verbunden ist.

9. Kappenanordnung in Kombination mit einer Medikamentenabgabe-Haupteinheit nach Anspruch 7 oder 8, wobei die Patroneneinheit (200) ferner dritte Kupplungsmittel (216) umfasst und die Kappe ferner Kappenkupplungsmittel (243) umfasst, die so ausgelegt sind, dass sie mit den dritten Kupplungsmitteln der Patroneneinheit lösbar in Eingriff treten können.

10. Kappenanordnung (200) in Kombination mit einer Medikamentenabgabe-Haupteinheit (100) nach einem der Ansprüche 2, 3, 7-9, wobei:
- die Kolbenstange (131, 132) in einer proximalen Ausgangsposition angeordnet sein kann,
- der Kolben (221) in einer vollständig gefüllten Patrone axial innerhalb eines anfänglich vordefinierten Positionsbereichs angeordnet ist, und
- die Patrone (220) relativ zur Kappe (240) aus einer Ausgangs- in eine Betriebsposition axial beweglich ist,
wobei die Kappenanordnung (200) mit der Patrone in der Ausgangsposition mit der Medikamentenabgabe-Haupteinheit (100) verbunden werden kann aus:
(i) einer anfänglichen axialen Position, in der die Kolbenstange (131, 132) mit dem Patronenkolben (221) in Eingriff steht, über
(ii) eine axiale Zwischenposition, in der die Patrone (220) in eine äußerste distale Position in der Kappe (240) und in Eingriff mit der Nadelanordnung (235) bewegt worden ist, wobei das proximale Nadelende (232) dadurch die Patronendichtung (222) durchdringt, und in
(iii) eine axiale Betriebsposition, in der der Patronenkolben (221) in der Patrone distal bewegt wurde, um dadurch eine Medikamentenmenge über die Hohlnadel (231,232) in das Reservoir (245) auszustoßen.

11. Kappenanordnung (200) in Kombination mit einer Medikamentenabgabe-Haupteinheit (100) nach einem der Ansprüche 2, 3, 7-10, wobei die Medikamentenabgabe-Haupteinheit ferner eine Dosiserfassungsschaltung (140) umfasst, die so ausgelegt ist, dass sie die Variation eines Parameters in der Kappenanordnung oder der Medikamentenabgabe-Haupteinheit bestimmt, der die Größe eines Satzes und/oder eine ausgestoßene Medikamentenmenge anzeigt.

12. Kappenanordnung (200) in Kombination mit einer Medikamentenabgabe-Haupteinheit (100) nach Anspruch 11, wobei es sich bei dem ermittelten Parameter um die Drehung einer Komponente im Dosiseinstellungs- und -ausstoßmechanismus (130) handelt.

13. Kappenanordnung (200) in Kombination mit einer Medikamentenabgabe-Haupteinheit (100) nach Anspruch 11 oder 12, ferner umfassend drahtlose Kommunikationsmittel, die es ermöglichen, erfasste Daten an eine externe Vorrichtung zu übermitteln.

14. Kappenanordnung (200) in Kombination mit einer Medikamentenabgabe-Haupteinheit (100) nach einem der Ansprüche 2, 3, 7-13, wobei die abgedichtete Kammer (245) in das Innere der Kappe entlüftet (248) wird.

## Revendications

1. Ensemble de capuchon (201) adapté pour être fixé à une unité principale d'administration de médicament (100), comprenant :
- une cartouche (220) remplie d'un médicament et définissant un axe général, la cartouche comprenant un piston mobile axialement (221) et une partie distale avec un joint pénétrable par une aiguille (222),
- un ensemble d'aiguille (230) comprenant une aiguille creuse ayant une extrémité distale d'aiguille de perçage cutané (231) et une extrémité proximale d'aiguille de perçage de joint (232), et
- un capuchon (240) recevant l'ensemble d'aiguille et au moins la partie distale de la cartouche,
dans lequel la cartouche (220) est agencée pour être déplacée distalement en engagement avec l'ensemble d'aiguille (230) entre un état initial dans lequel l'extrémité d'aiguille proximale (232) est agencée distalement du joint de cartouche (222) et un état opérationnel dans lequel l'extrémité d'aiguille proximale a pénétré le joint de cartouche,
**caractérisé en ce que**
- le capuchon comprend une chambre scellée (245) avec une barrière pénétrable par aiguille (246) adaptée pour recevoir l'extrémité distale de l'aiguille (231), et
- la chambre scellée est adaptée pour recevoir un médicament à partir de la cartouche via l'aiguille creuse.

2. Ensemble de capuchon selon la revendication 1, en combinaison avec une unité principale d'administration de médicament (100) comprenant :
- un logement (110),
- une tige de piston (131, 132), et
- un mécanisme de réglage et d'expulsion de dose (130) adapté pour déplacer la tige de piston distalement en correspondance avec une dose réglée,
dans lequel la cartouche (220) et l'unité principale d'administration de médicament (100) sont adaptées pour être connectés de manière amovible l'une à l'autre, la tige de piston étant agencée pour engager et déplacer le piston de cartouche distalement pour expulser alors une quantité de médicament à partir de la cartouche via l'aiguille creuse, le capuchon étant monté de manière amovible par rapport à la cartouche.

3. Ensemble de capuchon en combinaison avec une unité principale d'administration de médicament selon la revendication 2, dans lequel la cartouche (220) est agencée pour être déplacée distalement en engagement avec l'ensemble d'aiguille (230) correspondant à l'état opérationnel lorsque l'ensemble de capuchon dans l'état initial et l'unité principale d'administration de médicament sont connectés l'un à l'autre.

4. Ensemble de capuchon selon la revendication 1, comprenant en outre une unité de cartouche (200), l'unité de cartouche comprenant :
- la cartouche (220),
- un premier moyen de couplage (227) d'unité de cartouche,
- un deuxième moyen de couplage (218) d'unité de cartouche, et
- un moyen de guidage (215, 216) d'unité de cartouche,
le capuchon (240) comprenant en outre un moyen de guidage de capuchon (242) adapté pour engager le guidage d'unité de cartouche afin de fournir un mouvement axial guidé entre eux,
dans lequel l'ensemble d'aiguille (230) comprend un moyen de couplage d'aiguille (237) adapté pour engager le premier moyen de couplage (227) d'unité de cartouche correspondant à l'état opérationnel dans lequel l'extrémité d'aiguille proximale a pénétré le joint de cartouche, et
dans lequel l'unité de cartouche (200) peut être déplacée axialement à partir d'une position proximale initiale dans laquelle l'extrémité d'aiguille proximale (232) est agencée distalement du joint de cartouche (222), à une position opérationnelle distale dans laquelle l'extrémité d'aiguille proximale a pénétré le joint de cartouche et le moyen de couplage d'aiguille a engagé le premier moyen de couplage d'unité de cartouche.

5. Ensemble de capuchon selon la revendication 4, dans lequel l'ensemble d'aiguille (230) a une position axiale initiale dans laquelle l'extrémité d'aiguille distale est agencée dans le réservoir.

6. Ensemble de capuchon selon la revendication 4, dans lequel :
- l'ensemble d'aiguille (230) est agencé de manière mobile axialement par rapport au capuchon entre une position initiale dans laquelle l'extrémité distale d'aiguille (231) est agencée proximalement du réservoir (245), et une position opérationnelle dans laquelle l'extrémité d'aiguille distale est agencée dans le réservoir, et
- l'ensemble aiguille (230) est déplacé à partir de la position initiale à la position opérationnelle lorsque l'unité de cartouche est déplacée à partir de la position initiale à la position opérationnelle.

7. Ensemble de capuchon selon l'une quelconque des revendications 4-6, en combinaison avec une unité principale d'administration de médicament (100) comprenant :
- un logement (110),
- une tige de piston (131, 132),
- un mécanisme de réglage et d'expulsion de dose (130) adapté pour déplacer la tige de piston distalement correspondant à une dose réglée, et
- un moyen de couplage d'unité principale (128),
dans lequel l'ensemble de capuchon (201) et l'unité principale d'administration de médicament (100) sont adaptés pour être connectés de manière amovible l'un à l'autre, la tige de piston (131, 132) étant agencée pour engager et déplacer le piston de cartouche (221) distalement pour expulser alors une quantité de médicament à partir de la cartouche via l'aiguille creuse, et
dans lequel le moyen de couplage de l'unité principale (128) est adapté pour engager de manière amovible le deuxième moyen de couplage d'unité de cartouche (218).

8. Ensemble de capuchon en combinaison avec une unité principale d'administration de médicament selon la revendication 7, dans lequel l'unité de cartouche (200) est agencée pour être déplacée distalement dans la position opérationnelle distale dans laquelle l'extrémité d'aiguille proximale (232) a pénétré le joint de cartouche (222) et le moyen de couplage d'aiguille a engagé le premier couplage d'unité de cartouche lorsque l'ensemble de capuchon est connecté à l'unité principale d'administration de médicament.

9. Ensemble de capuchon en combinaison avec une unité principale d'administration de médicament selon la revendication 7 ou 8, dans lequel l'unité de cartouche (200) comprend en outre un troisième moyen de couplage (216), et le capuchon comprenant en outre un moyen de couplage de capuchon (243) adapté pour engager de manière amovible le troisième moyen de couplage d'unité de cartouche.

10. Ensemble de capuchon (200) en combinaison avec une unité principale d'administration de médicament (100) selon l'une quelconque des revendications 2, 3, 7-9, dans lequel :
- la tige de piston (131, 132) peut être agencée dans une position initiale la plus proximale,
- le piston (221) dans une cartouche entièrement remplie axialement est agencé dans une plage de position prédéfinie initiale, et
- la cartouche (220) est mobile axialement par rapport au capuchon (240) à partir d'une position initiale à une position opérationnelle,
dans lequel l'ensemble de capuchon (200) avec la cartouche dans la position initiale peut être connecté à l'unité principale d'administration de médicament (100) à partir de :
(i) une position axiale initiale dans laquelle la tige de piston (131, 132) engage le piston de cartouche (221), via
(ii) une position axiale intermédiaire dans laquelle la cartouche (220) a été déplacée vers une position la plus distale dans le capuchon (240) et en engagement avec l'ensemble d'aiguille (235), l'extrémité d'aiguille proximale (232) pénétrant alors le joint de cartouche (222), et vers
(iii) une position axiale opérationnelle dans laquelle le piston de cartouche (221) a été déplacé distalement dans la cartouche pour expulser alors une quantité de médicament dans le réservoir (245) via l'aiguille creuse (231,232).

11. Ensemble de capuchon (200) en combinaison avec une unité principale d'administration de médicament (100) selon l'une quelconque des revendications 2, 3, 7-10, dans lequel l'unité principale d'administration de médicament comprend en outre un circuit de capture de dose (140) adapté pour déterminer une variation d'un paramètre dans l'ensemble de capuchon ou dans l'unité principale d'administration de médicament étant indicative de la taille d'un ensemble et/ ou d'une quantité de médicament expulsée.

12. Ensemble de capuchon (200) en combinaison avec une unité principale d'administration de médicament (100) selon la revendication 11, dans lequel le paramètre détecté est une rotation d'un composant dans le mécanisme de réglage et d'expulsion de dose (130).

13. Ensemble de capuchon (200) en combinaison avec une unité principale d'administration de médicament (100) selon la revendication 11 ou 12, comprenant en outre un moyen de communication sans fil permettant que des données capturées soient transmises à un dispositif externe.

14. Ensemble de capuchon (200) en combinaison avec une unité principale d'administration de médicament (100) selon l'une quelconque des revendications 2, 3, 7-13, dans lequel la chambre scellée (245) donne (248) sur l'intérieur du capuchon.
